# FASCICULE DE BREVET EUROPEEN

(11) **EP 2 091 905 B1**
(45) Date de publication et mention de la délivrance du brevet: **13.05.2015**
(21) Numéro de dépôt: 07822844.2
(22) Date de dépôt: 23.11.2007
(51) Int. Cl.: C07C 51/08, C07C 67/08, C07C 55/12, C07C 53/128, C07C 69/42, C07C 69/24

(54) **PROCEDE DE TRANSFORMATION DE COMPOSES NITRILES EN ACIDES CARBOXYLIQUES ET ESTERS CORRESPONDANTS**
VERFAHREN ZUR UMWANDLUNG VON NITRILVERBINDUNGEN IN ENTSPRECHENDE CARBONSÄUREN UND ESTER
METHOD FOR CONVERTING NITRILE COMPOUNDS INTO CORRESPONDING CARBOXYLIC ACIDS AND ESTERS

(30) Priorité: 24.11.2006 FR 0610302
(43) Date de publication de la demande: 26.08.2009
(73) Titulaire: Rhodia Opérations, 93306 Aubervilliers (FR)
(72) Inventeur: BUISINE, Olivier, 69230 Saint-Genis Laval (FR); LECONTE, Philippe, 68150 RIBEBEAUVILLE (FR); AGATI, Mélanie, 69440 MORNANT (FR)
(74) Mandataire: Jacobson, Claude
(86) Numéro de dépôt international: PCT/EP2007/062748
(87) Numéro de publication internationale: WO 2008/062058

(56) Documents cités:
- GB-A- 339 235
- GB-A- 970 953
- GB-A- 1 122 448
- GB-A- 1 261 949
- US-A- 3 377 378
- US-A- 3 492 345
- US-A- 3 567 749
- US-A- 4 028 406
- US-A- 4 236 018

## Description

La présente invention concerne un procédé de transformation de composés hydrocarbonés comprenant au moins une fonction nitrile en composés comprenant au moins une fonction carboxylique et également un procédé d'obtention de composés esters à partir de ces composés carboxyliques ainsi obtenus.

L'invention concerne plus particulièrement un procédé de transformation des composés nitriles obtenus comme produits et sous produits dans l'hydrocyanation du butadiène tel que, par exemple, le méthyl-butyronitrile, le valéronitrile, le 2-méthyl-2-butènenitrile, le 2-pentènenitrile, 3-pentènenitrile, 4-pentènenitrile,2-méthyl-3-butènenitrile, et les dinitriles tels que le méthyl-glutaronitrile, l'éthyl-succinonitrile et éventuellement l'adiponitrile ou le mélange de ces trois composés en composés diacides d'une part et composés diesters d'autre part.

Le procédé de fabrication d'adiponitrile par hydrocyanation du butadiène est exploité industriellement depuis plusieurs dizaines d'années. Ce procédé présente une sélectivité élevée en adiponitrile, intermédiaire chimique important pour la synthèse de l'hexaméthylène diamine ou du caprolactame et la fabrication des polyamides.

Toutefois, ce procédé produit également des composés dinitriles ramifiés tels que le méthyl-glutaronitrile, l'éthyl-succinonitrile notamment qui sont séparés et récupérés par distillation.

En général, cette récupération des composés dinitriles ramifiés permet de produire un mélange contenant majoritairement du méthyl-glutaronitrile avec de l'éthyl-succinonitrile et de l'adiponitrile.

Plusieurs solutions ont été proposées pour valoriser ces sous-produits ou mélanges. Une de celles-ci consiste à hydrogéner les composés dinitriles en amines primaires notamment pour produire de la méthylpentaméthylènediamine (MPMD), utilisée comme monomère pour la fabrication de polyamides particuliers. Ce procédé requiert des étapes de purification soit du méthyl-glutaronitrile soit de la méthylpentaméthylènediamine. Dans l'industrie, ces sous-produits sont souvent détruits par combustion avec valorisation sous forme de vapeur ou d'énergie mais avec production d'effluents gazeux contenant du CO₂, et des oxydes d'azote.

US 4,028,406 décrit la préparation d'acide de thiazolidine-4-carboxylique à partir de thiazolidine-4-carbonenitrile par hydrolyse en deux étapes. US 4,236,018 décrit la préparation d'acide cyclopropane-carboxylique à partir de cyclopropane-nitrile par réaction avec de l'eau et de l'acide sulfurique.

Il existe donc un besoin et une demande importante de trouver de nouvelles voies de valorisation et transformation de ces composés nitriles ou des mélanges en composés chimiques valorisables et économiquement intéressants.

Un des buts de la présente invention est notamment de proposer un procédé permettant de transformer ces composés nitriles en composés carboxyliques d'une part, utilisables notamment comme intermédiaires chimiques tels que, par exemple, monomères pour la fabrication de polyuréthanes et polyamides, ou en composés diesters d'autre part, utilisables notamment comme solvant.

A cet effet, l'invention propose un procédé de transformation de composés comprenant au moins une fonction nitrile et comprenant de 4 à 10 atomes de carbone en composés comprenant au moins une fonction carboxylique consistant à hydrater les fonctions nitriles en fonction amide par réaction avec l'eau en présence d'un acide fort minéral, puis à hydrolyser les fonctions amides en fonctions carboxyliques par réaction avec l'eau et un acide fort minéral.

Selon l'invention, la réaction d'hydratation est réalisée sous agitation, en utilisant de 1 à 1,5 moles d'eau par mole de fonction nitrile à hydrater en présence d'un acide fort minéral à une température permettant de maintenir le milieu réactionnel à l'état liquide, avantageusement à une température supérieure ou égale à 90°C et préférentiellement à une température comprise entre 90°C et 180°C.

L'acide minéral fort est avantageusement choisi dans le groupe comprenant l'acide chlorhydrique gazeux, l'acide phosphorique, l'acide sulfurique ou analogue. L'acide est ajouté avantageusement sous forme pure ou très concentrée pour contrôler la quantité d'eau ajoutée dans le milieu réactionnel.

En outre, la réaction d'hydrolyse est également réalisée sous agitation, en utilisant de 1 à 10 moles d'eau par mole de fonction amide à hydrolyser, de préférence de 1 à 5 moles d'eau et encore plus avantageusement de 1 à 2 moles d'eau et une quantité d'acide fort minéral exprimée en proton et correspondant à au moins 1 mole de proton par mole d'amide à hydrolyser, la température du milieu réactionnel étant déterminée pour maintenir le milieu réactionnel à l'état liquide, avantageusement à une température supérieure ou égale à 90°C et préférentiellement à une température comprise entre 90°C et 180°C.

Les acides forts minéraux convenables sont ceux décrits dans la liste ci-dessus.

Dans un autre mode de réalisation de l'invention, l'eau nécessaire pour les réactions d'hydratation des fonctions nitriles et l'hydrolyse des fonctions amides est ajoutée en début de réaction d'hydratation. Ainsi, la quantité d'eau ajoutée est avantageusement comprise entre 2 et 10 moles d'eau (bornes comprises) par fonction nitrile à hydrater, de préférence de 2 à 3 moles d'eau par fonction nitrile.

Selon l'invention, après la fin de la réaction d'hydrolyse, l'agitation du milieu réactionnel est arrêtée pour permettre audit milieu de décanter tout en maintenant la température à une valeur telle que le milieu réactionnel soit maintenu à l'état liquide.

On obtient une séparation en deux phases dont la phase supérieure comprend essentiellement l'acide carboxylique formé.

Ainsi, selon le procédé de l'invention, il est possible de récupérer et séparer le composé carboxylique selon une technologie simple à savoir la décantation et séparation de phases.

Cette récupération et séparation de phases sont obtenues par la réalisation de l'hydratation et l'hydrolyse des fonctions nitriles en milieu acide et par l'utilisation d'une quantité déterminée d'eau dans les deux étapes et la présence d'un sel dans le milieu d'hydrolyse.

L'eau et le sel d'ammonium produits sont également aisément récupérables car ils constituent la phase inférieure du milieu réactionnel. Dans un mode de réalisation particulier de l'invention, de l'eau peut être additionnée dans la phase inférieure, avantageusement après séparation de la phase supérieure, pour favoriser la cristallisation du sel d'ammonium à température ambiante (15°C-30°C). La phase aqueuse après séparation du sel cristallisé, qui peut contenir une faible quantité d'acides carboxyliques et éventuellement forts, peut être avantageusement recyclée à l'étape d'hydratation des composés nitriles.

Selon un mode particulier de l'invention, le composé carboxylique récupéré, est utilisé avantageusement comme matière première pour la fabrication d'esters, par réaction avec un alcool, avantageusement en ajoutant une quantité d'alcool stoechiométrique ou voisine de la stoechiométrie, c'est à dire environ deux moles d'alcool par mole de diacide.

Cette réaction peut être réalisée sans addition complémentaire de composés acides comme catalyseur. En effet, la quantité de composés acides (acide fort et/ou sel d'ammonium) présente dans la phase supérieure du milieu réactionnel obtenu à l'étape d'hydrolyse peut être suffisante pour catalyser la réaction d'estérification. Ainsi, l'estérification peut être réalisée par mélange de la phase supérieure obtenue après décantation dans le procédé de fabrication d'acide carboxylique avec un alcool et maintien du milieu réactionnel à une température permettant avantageusement d'avoir un reflux de l'alcool engagé. Toutefois, il est également possible de travailler à une température légèrement inférieure à la température définie ci-dessus.

Toutefois, sans sortir du cadre de l'invention, il est possible d'ajouter une quantité déterminée de catalyseur acide pour réaliser l'étape d'estérification. Ce catalyseur peut être constitué par un acide minéral fort et/ou par une partie de la phase inférieure aqueuse contenant le sel acide obtenu après l'étape d'hydrolyse.

La présence d'un sel dans le milieu d'estérification favorise la décantation de l'eau et donc la formation de l'ester.

Toutefois, pour obtenir un taux de transformation élevé, voisin de 100% du diacide en diester, il est avantageux, soit après séparation de la phase aqueuse décantée ou sans séparation de cette phase aqueuse, d'éliminer l'eau formée par addition en continu d'alcool et distillation d'un mélange eau/alcool jusqu'à élimination quasi totale de l'eau formée. L'alcool distillé avec l'eau peut être séparé de l'eau, par exemple par décantation, et recyclé dans la réaction d'estérification.

Ainsi, cette réaction peut être réalisée par passage sur une résine sulfonique acide à une température comprise entre 40 et 150°C. Le milieu issu de la colonne contenant la résine est ensuite distillé pour séparer l'alcool n'ayant pas réagi et l'eau formé.

Elle peut également être mise en oeuvre dans une colonne à distiller réactive permettant de réaliser la réaction d'estérification simultanément à l'extraction de l'eau formée par distillation. Ainsi, il est possible de déplacer l'équilibre pour obtenir un taux de transformation voisin de 100%.

Les alcools convenables sont, par exemple, les alcools aliphatiques ramifiés ou non, cycliques ou non, pouvant comporter un noyau aromatique et pouvant comprendre de 1 à 20 atomes de carbone. A titre d'exemples préférés, on peut citer les alcools suivants, méthanol, propanol, isopropanol, alcool benzylique, éthanol, n-butanol, isobutanol, cyclohexanol, hexanol, isooctanol, 2-éthyl hexanol, pentanols, méthyl-butanol ou analogues.

Le milieu contenant les diesters est ensuite, avantageusement, distillé dans une installation comprenant une étape d'étêtage et d'équeutage. Cette distillation peut être réalisée dans une seule colonne avec récupération des diesters sous forme d'une fraction intermédiaire.

Le procédé de l'invention s'applique aux composés comprenant une ou plusieurs fonctions nitriles.

Dans un mode de réalisation préféré, il s'applique plus particulièrement à la transformation des composés comprenant deux fonctions nitriles en acides dicarboxyliques puis diesters.

On peut citer comme composés nitriles convenables pour le procédé de l'invention, le méthyl-butyronitrile, le valéronitrile, le 2-méthyl-2-butènenitrile, le 2-pentènenitrile, le 3-pentènenitrile, le 4-pentènenitrile, le 2-méthyl-3-butènenitrile, et les dinitriles tels que le méthyl-2 butyronitrile, le méthyl- gultaronitrile, l'éthyl-succinonitrile, le succinonitrile, le glutaronitrile, l'adiponitrile ou analogues ou un mélange d'au moins deux desdits composés.

Selon un mode particulièrement privilégié, les composés nitriles convenables pour l'invention sont les composés dinitriles formés dans le procédé d'hydrocyanation du butadiène, plus particulièrement les composés dinitriles ramifiés tels que le méthyl-glutaronitrile et l'éthyl- succinonitrile, seuls ou mélange.

Encore plus préférentiellement, la matière première utilisée pour la mise en oeuvre du procédé de l'invention est constituée par la fraction de distillation comprenant les composés dinitriles ramifiés formés dans le procédé d'hydrocyanation du butadiène, et notamment récupérée dans l'étape de séparation et purification de l'adiponitrile.

Ainsi, cette matière première comprend principalement du méthyl glutaronitrile, de l'éthylsuccinonitrile et de l'adiponitrile. Le méthyl glutaronitrile est majoritaire dans ce mélange.

Le procédé de l'invention permet donc de produire un mélange de composés dicarboxyliques et un mélange de composés diesters.

Il est bien entendu possible de séparer les différents composés de ce mélange par les techniques habituelles telles que distillation, cristallisation, extraction liquide/liquide. Toutefois, dans de nombreuses applications, le mélange de composés peut être utilisé, sans séparation.

Ainsi, le mélange de diesters présente des propriétés de solvant très intéressantes et est utilisé en remplacement ou en mélange avec des solvants usuels tels que la méthylbutyrolactone, le N-méthylpyrrolidone, l'acétonitrile ou analogue.

A titre d'exemple, les diesters ainsi obtenus peuvent être utilisés dans de nombreuses applications telles que comme solvant dans les peintures, vernis, laques, l'industrie du revêtement de surfaces ou tout autre articles comme les câbles par exemple, l'industrie des encres des lubrifiants pour textiles, les liants et résines pour noyaux de moules de fonderie, les produits nettoyants, les formulations cosmétiques. Ils peuvent également être utilisés comme matières premières pour certaines réactions chimiques, dans les compositions de traitement des sols et végétaux.

Plus généralement, ils peuvent être utilisés seul ou dans une formulation, comme solvant de nettoyage, décapage, dégraissage dans toute activité industrielle ou domestique. Ces diesters peuvent également être utilisés comme plastifiants de certaines matières plastiques ou comme monomères pour la fabrication de polymères.

De même les diacides obtenus sont avantageusement utilisés sous forme de mélange dans, par exemple, la fabrication de polyuréthanne, polyesters, polyamides ou analogues. Le procédé de l'invention peut être mis en oeuvre de manière discontinue ou continue. Les acides carboxyliques et/ou diesters peuvent être purifiés par les techniques usuelles telles que, par exemple, traitement sur résine échangeuse d'ions, traitement avec le charbon actif, distillation, cristallisation, extraction liquide/liquide.

D'autres détails et avantages de l'invention apparaîtront plus clairement au vu des exemples donnés ci-dessous à titre d'illustration, uniquement.

### Exemple 1 :

Dans un réacteur de 1 litre, muni d'une agitation et surmonté d'un réfrigérant, on charge sous azote à température ambiante, 50 g d'eau puis on coule 220,43 g d'H₂SO₄ 98%. La température est ensuite amenée à 105°C.
108,68 g de MGN sont alors introduits en 2 h et la température du milieu est maintenue à 120°C.
Après maintien 15 min en température, 58,5 g d'eau sont coulés sur le milieu homogène en 10 min puis la température est portée à 125°C et maintenue pendant 6 h.
L'agitation est arrêtée et deux phases liquides décantent très rapidement.

Le dosage potentiométrique à la soude de la phase inférieure montre que le rapport molaire (NH₄)HSO₄ / (H₂SO₄ + (NH₄)HSO₄) correspond à une conversion complète du MGN. Un dosage par chromatographie en phase liquide (CLHP) peut également être réalisé.

On refroidi le milieu réactionnel à 90°C, et la phase supérieure est séparée de la phase aqueuse et transférée dans un réacteur de 500 ml. 147,47 g d'acide méthyl-2-glutarique brutes sont ainsi obtenus.

64 g de méthanol sont ajoutés sur le diacide et la température est maintenue à 60°C sous agitation pendant 1 h L'agitation est arrêtée et une couche aqueuse de 20 g environ décante. Cette dernière est séparée et l'analyse de la couche supérieure montre que plus de 60% des fonctions acide ont été transformées en fonctions ester. L'estérification peut-être complétée par addition de méthanol. On peut également séparer les composés diesters par distillation du milieu réactionnel et recycler les monoesters dans le milieu d'estérification pour augmenter le taux de transformation en diesters.

### Exemple 2 :

Dans un réacteur de 1 litre, muni d'une agitation et surmonté d'un réfrigérant, on charge sous azote à température ambiante, 98,3 g d'eau (5,9 moles) puis on coule 379,2 g d'acide sulfurique à 98% (3,8 moles). La température est ensuite amenée à 105°C.

300 g de 2-méthylbutyronitrile sont alors introduits en 4 h et la température du milieu est maintenue une heure supplémentaire à 105°C. L'analyse du milieu réactionnel indique que la totalité des fonctions nitrile ont réagit. 89 g d'eau sont ajoutés et la température est portée à 120°C et maintenue pendant 15h. La température est alors abaissée à 70°C, l'agitation est arrêtée et deux phases liquides décantent très rapidement. Une analyse potentiométrique à la soude de la phase inférieure montre que le rapport molaire (NH₄)HSO₄ / (H₂SO₄ + (NH₄)HSO₄) correspond à une conversion complète du 2-méthylbutyronitrile en acide carboxylique correspondant. Les phases sont séparées par décantation à cette température. On obtient ainsi 492g de phase aqueuse et 366g de couche organique constituée d'acide 2-méthylbutyrique.

329g d'éthanol (7,1 moles) et 18 g d'acide sulfurique (0,2mole) sont ajoutée à la phase organique et la température est maintenue à 80°C pendant 2 heures. L'analyse du milieu indique que 84% des fonctions acides ont été estérifiées.

La température est ensuite abaissée à 20°C, et du carbonate de sodium est ajoutée sous agitation pour neutraliser l'acide.

L'agitation est ensuite arrêtée et de l'eau est ajoutée pour obtenir deux phases liquides qui décantent. On obtient une phase aqueuse et 620g de phase organique contenant le 2-méthylbutyrate d'éthyle qui est ensuite purifié par distillation.

### Exemple 3 :

Dans un réacteur de 1 litre, muni d'une agitation et surmonté d'un réfrigérant, on charge sous azote à température ambiante, 98,3 g d'eau (5,9 moles) puis on coule 379,2 g d'acide sulfurique à 98% (3,8 moles). La température est ensuite amenée à 105°C.

300 g de 2-méthylbutyronitrile sont alors introduits en 4 h et la température du milieu est maintenue une heure supplémentaire à 105°C. L'analyse du milieu réactionnel indique que la totalité des fonctions nitrile ont réagit. 89 g d'eau sont ajoutés et la température est amenée à 120°C et maintenue pendant 15h. La température est alors abaissée à 70°C, l'agitation est arrêtée et deux phases liquides décantent très rapidement. Une analyse potentiométrique à la soude de la phase inférieure montre que le rapport molaire (NH₄)HSO₄ / (H₂SO₄ + (NH₄)HSO₄) correspond à une conversion complète du 2-méthylbutyronitrile en acide 2-méthylbutyrique. Les phases sont séparées par décantation à cette température. On obtient ainsi 492g de phase aqueuse et 367g de couche organique constituée d'acide valérique.

197g d'éthanol (4,3 moles) et 18 g d'acide sulfurique (0,2mole) sont ajoutée et la température est maintenue à 80°C pendant 2 heures puis ramenée à 20°C. L'agitation est arrêtée et une couche aqueuse de 62g décante. Celle-ci est éliminée et 18g d'acide sulfurique (0,2mole) sont ajoutée à la couche organique sous agitation. La température est maintenue à 80°C pendant deux heures puis ramenée à 20°C. Du carbonate de sodium est ajouté pour neutraliser l'acide. Après 30 minutes d'agitation, celle-ci est arrêtée et de l'eau est ajoutée pour obtenir deux phases qui décantent. Une analyse de la couche organique indique que 82% des fonctions nitrile de départ sont converties en ester éthylique. La phase organique de 497g est ensuite purifiée par distillation.

## Revendications

1. Procédé de fabrication d'acides carboxyliques comprenant de 4 à 10 atomes de carbone par hydratation des fonctions nitriles d'un composé organique en fonction amide et hydrolyse des fonctions amides en fonctions carboxyliques, **caractérisé en ce que** :
➢ L'étape d'hydratation est réalisée par réaction avec l'eau en présence d'un acide fort minéral, en utilisant de 1 à 1,5 moles d'eau par mole de fonction nitrile à hydrater en présence d'un acide fort minéral à une température permettant de maintenir le milieu réactionnel à l'état liquide
➢ L'étape d'hydrolyse est réalisée, sous agitation, en utilisant de 1 à 10 moles d'eau par mole de fonction amide à hydrolyser et une quantité d'acide fort minéral exprimée en proton et correspondant à au moins 1 mole de proton par mole d'amide à hydrolyser, la température du milieu réactionnel étant déterminée pour maintenir le milieu réactionnel à l'état liquide et **en ce que**
➢ L'acide carboxylique formé est récupéré par maintien du milieu réactionnel, sans agitation, à une température supérieure à la température de fusion de l'acide carboxylique et/ou du sel formé par décantation du milieu réactionnel, la phase supérieure contenant l'acide carboxylique étant séparée.

2. Procédé selon la revendication 1, **caractérisé en ce que** la quantité d'eau utilisée dans l'étape d'hydrolyse est comprise entre 1 et 5 moles d'eau par mole de fonction amide à hydrolyser, de préférence entre 1 et 2 moles d'eau.

3. Procédé selon l'une des revendications 1 ou 2, **caractérisé en ce que** l'eau nécessaire pour les réactions d'hydratation et d'hydrolyse est ajouté à l'étape d'hydratation selon une quantité d'eau comprise entre 2 et 10 moles d'eau, de préférence de 2 à 3 moles d'eau par fonction nitrile à hydrater.

4. Procédé selon l'une des revendications 1 à 3, **caractérisé en ce que** le composé comprenant des fonctions nitriles est choisi dans le groupe comprenant le méthyl-butyronitrile, le valéronitrile, le 2-méthyl-2-butènenitrile, le 2-pentènenitrile, le 3-pentènenitrile, le 4-pentènenitrile, le 2-méthyl-3-butènenitrile, et les dinitriles tels que le le méthyl-2 butyronitrile, le méthyl- gultaronitrile, l'éthyl-succinonitrile, le succinonitrile, le glutaronitrile, l'adiponitrile, ou analogues ou un mélange d'au moins deux desdits composés

5. Procédé selon l'une des revendications 1 à 4, **caractérisé en ce que**, le composé comprenant des fonctions nitriles est un mélange de méthyl-glutaronitrile, éthyl-succinonitrile, et d'adiponitrile.

6. Procédé selon la revendication 5, **caractérisé en ce que** ledit mélange constitue la fraction de tête de la purification ou séparation de l'adiponitrile par distillation dans le procédé de fabrication de l'adiponitrile par hydrocyanation du butadiène.

7. Procédé selon l'une des revendications 5 ou 6, **caractérisé en ce que** l'étape d'hydratation est réalisé à une température comprise entre 90°C et 180°C et l'étape d'hydrolyse à une température comprise entre 90°C et 180°C

8. Procédé selon l'une des revendications 1 à 7, **caractérisé en ce que** l'acide minéral est choisi dans le groupe comprenant l'acide chlorhydrique gazeux, l'acide phosphorique, l'acide sulfurique ou analogue.

9. Procédé selon la revendication 8, **caractérisé en ce que** l'acide minéral est ajouté sous forme pure ou concentrée.

10. Procédé selon l'une des revendications précédentes, **caractérisé en ce qu'**il comprend une étape d'estérification des acides carboxyliques contenus dans la phase supérieure en esters par réaction avec un alcool.

11. Procédé selon la revendication 10, **caractérisé en ce que** l'étape d'estérification est réalisée par mélange de la phase supérieure séparée avec un alcool et maintien du milieu réactionnel à une température permettant d'obtenir un reflux de l'alcool.

12. Procédé selon la revendication 10 ou 11, **caractérisé en ce que** la quantité d'alcool mélangée correpond à 2 mole d'alcool par mole de diacide.

13. Procédé selon l'une des revendications 10 à 12, **caractérisé en ce que** une estérification totale du diacide est réalisée par élimination de l'eau formée obtenue par addition en continu d'alcool et distillation d'un mélange eau/alccol.

14. Procédé selon l'une des revendications 10 à 13, **caractérisé en ce qu'**un acide minéral est ajouté comme catalyseur.

15. Procédé selon l'une des revendications 10 à 14, **caractérisé en ce que** l'alcool est choisi dans le groupe comprenant les alcools aliphatiques ramifiés ou non, cycliques ou non, pouvant comporter un noyau aromatique et pouvant comprendre de 1 à 20 atomes de carbone.

16. Procédé selon la revendication 15, **caractérisé en ce que** l'alcool est choisi dans le groupe comprenant le méthanol, propanol, isopropanol, alcool benzylique, éthanol, n-butanol, isobutanol, cyclohexanol, hexanol, isooctanol, 2-éthyl hexanol, les isomères des pentanols, l'isobutanol.

17. Procédé selon l'une des revendications 10 à 16, **caractérisé en ce que** la réaction d'estérification est réalisée dans une colonne réactive avec élimination de l'eau.

18. Procédé selon l'une des revendications 10 à 16, **caractérisé en ce que** la réaction d'estérification est réalisée par passage du mélange composé carboxylique/alcool sur une résine sulfonique acide échangeuse d'ions.

19. Procédé selon l'une des revendications 10 à 18, **caractérisé en ce que** les esters formés sont récupérés et purifiés par distillation.

## Patentansprüche

1. Ein Verfahren zur Herstellung von Carbonsäuren, die 4-10 Kohlenstoffatome aufweisen, durch Hydratisierung von Nitrilgruppen einer organischen Verbindung zu Amidgruppen und Hydrolyse der Amidgruppen zu Carboxylgruppen, **dadurch gekennzeichnet, dass**:
➢ der Hydratisierungsschritt verwirklicht wird durch Reaktion mit Wasser in Gegenwart einer starken anorganischen Säure unter Verwendung von 1 bis 1,5 Mol Wasser pro Mol Nitrilgruppe, welche hydratisiert werden soll, in Gegenwart einer starken anorganischen Säure bei einer Temperatur, welche es erlaubt das Reaktionsmilieu in flüssigem Zustand zu halten,
➢ der Hydrolyseschritt verwirklicht wird unter Rühren unter Verwendung von 1 bis 10 Mol Wasser pro Mol Amidgruppe, welche hydrolysiert werden soll, und einer Menge von starker anorganischer Säure, welche in Protonen angegeben ist und wenigstens 1 Mol Protonen pro Mol Amid, welches hydrolysiert werden soll, entspricht, wobei die Temperatur des Reaktionsmilieus ermittelt wird, um das Reaktionsmilieu in flüssigem Zustand zu halten, und dass
➢ die gebildete Carbonsäure gesammelt wird durch Beibehaltung des Reaktionsmilieus, ohne Rühren, bei einer Temperatur, die größer ist als die Schmelztemperatur der gebildeten Carbonsäure und/oder des gebildeten Salzes, wobei durch Dekantieren von dem Reaktionsmilieu die obere Phase, welche die Carbonsäure enthält, abgetrennt wird.

2. Das Verfahren gemäß Anspruch 1, **dadurch gekennzeichnet, dass** die Menge von Wasser, welche im Hydrolyseschritt verwendet wird, zwischen 1 und 5 Mol Wasser, vorzugsweise zwischen 1 und 2 Mol Wasser, pro Mol Amidgruppe, welche hydrolysiert werden soll, aufweist.

3. Das Verfahren gemäß einem der Ansprüche 1 oder 2, **dadurch gekennzeichnet, dass** das Wasser, welches für die Hydratisierungsreaktion und die Hydrolysereaktion erforderlich ist, im Hydratisierungsschritt dazugegeben wird gemäß einer Menge von Wasser, welche zwischen 2 und 10 Mol Wasser, vorzugsweise zwischen 2 und 3 Mol Wasser, pro Nitrilgruppe, welche hydratisiert werden soll, aufweist.

4. Das Verfahren gemäß einem der Ansprüche 1 bis 3, **dadurch gekennzeichnet, dass** die Verbindung, welche Nitrilgruppen aufweist, aus der Gruppe, die Methylbutyronitril, Valeronitril, 2-Methyl-2-butennitril, 2-Pentennitril, 3-Pentennitril, 4-Pentennitril, 2-Methyl-3-butennitril und Dinitrile, wie z.B. 2-Methylbutyronitril, Methylglutaronitril, Ethylsuccinonitril, Succinonitril, Glutaronitril, Adipodinitril oder dergleichen oder ein Gemisch aus mindestens zwei der genannten Verbindungen aufweist, ausgewählt ist.

5. Das Verfahren gemäß einem der Ansprüche 1 bis 4, **dadurch gekennzeichnet, dass** die Verbindung, welche Nitrilgruppen aufweist ein Gemisch aus Methylglutaronitril, Ethylsuccinonitril und Adipodinitril ist.

6. Das Verfahren gemäß Anspruch 5, **dadurch gekennzeichnet, dass** das genannte Gemisch die Kopffraktion der Reinigung oder Trennung von Adiponitril durch Destillation in dem Verfahren zur Herstellung von Adiponitril durch die Hydrocyanierung von Butadien darstellt.

7. Das Verfahren gemäß einem der Ansprüche 5 oder 6, **dadurch gekennzeichnet, dass** der Hydratisierungsschritt bei einer Temperatur, welche zwischen 90°C und 180°C aufweist, und der Hydrolyseschritt bei einer Temperatur, welche zwischen 90°C und 180°C aufweist, verwirklicht wird.

8. Das Verfahren gemäß einem der Ansprüche 1 bis 7, **dadurch gekennzeichnet, dass** die anorganische Säure aus der Gruppe, die gasförmige Chlorwasserstoffsäure, Phosphorsäure, Schwefelsäure und dergleichen aufweist, ausgewählt ist.

9. Das Verfahren gemäß Anspruch 8, **dadurch gekennzeichnet, dass** die anorganische Säure in reiner oder konzentrierter Form dazugegeben wird.

10. Das Verfahren gemäß einem der vorhergehenden Ansprüche, **dadurch gekennzeichnet, dass** es einen Veresterungsschritt der Carbonsäuren, welche in der oberen Phase enthalten ist, zu Estern durch Reaktion mit einem Alkohol aufweist.

11. Das Verfahren gemäß Anspruch 10, **dadurch gekennzeichnet, dass** der Veresterungsschritt verwirklicht wird durch Vermischung der abgetrennten oberen Phase mit einem Alkohol und Beibehaltung des Reaktionsmilieus bei einer Temperatur, welche es zulässt einen Rückfluss des Alkohols zu erhalten.

12. Das Verfahren gemäß Anspruch 10 oder 11, **dadurch gekennzeichnet, dass** die Menge von Alkohol, welche vermischt wird, 2 Mol Alkohol pro Mol Disäure entspricht.

13. Das Verfahren gemäß einem der Ansprüche 10 bis 12, **dadurch gekennzeichnet, dass** eine vollständige Veresterung der Disäure durch Entfernen des gebildeten Wassers, erreicht durch kontinuierliche Zugabe von Alkohol und Destillation eines Wasser/Alkohol-Gemisches, verwirklicht wird

14. Das Verfahren gemäß einem der Ansprüche 10 bis 13, **dadurch gekennzeichnet, dass** eine anorganische Säure als Katalysator dazugegeben wird.

15. Das Verfahren gemäß einem der Ansprüche 10 bis 14, **dadurch gekennzeichnet, dass** der Alkohol aus der Gruppe, die verzweigte und nicht-verzweigte, cyclische und nicht-cyclische aliphatische Alkohole, welche einen aromatischen Ring aufweisen können und 1 bis 20 Kohlenstoffatome aufweisen können, aufweist, ausgewählt ist.

16. Das Verfahren gemäß Anspruch 15, **dadurch gekennzeichnet, dass** der Alkohol aus der Gruppe, die Methanol, Propanol, Isopropanol, Benzylalkohol, Ethanol, n-Butanol, Isobutanol, Cyclohexanol, Hexanol, Isooctanol, 2-Ethylhexanol, isomere von Pentanol, Isobutanol aufweist, ausgewählt ist.

17. Das Verfahren gemäß einem der Ansprüche 10 bis 16, **dadurch gekennzeichnet, dass** die Veresterungsreaktion in einer Reaktionskolonne mit Entfernen von Wasser verwirklicht ist.

18. Das Verfahren gemäß einem der Ansprüche 10 bis 16, **dadurch gekennzeichnet, dass** die Veresterungsreaktion durch Hindurchtreten des Carbonsäure/Alkohol-Gemischs durch ein Sulfonsäure-Ionentauscherharz verwirklich ist.

19. Das Verfahren gemäß einem der Ansprüche 10 bis 18, **dadurch gekennzeichnet, dass** die gebildeten Ester durch Destillation gesammelt und gereinigt werden.

## Claims

1. Process for the manufacture of carboxylic acids comprising from 4 to 10 carbon atoms by hydration of the nitrile functional groups of an organic compound to give amide functional groups and hydrolysis of the amide functional groups to give carboxyl functional groups, **characterized in that**:
➢ the hydration stage is carried out by reaction with water in the presence of a strong inorganic acid by using from 1 to 1.5 mol of water per mole of nitrile functional group to be hydrated in the presence of a strong inorganic acid at a temperature which makes it possible to maintain the reaction medium in the liquid state;
➢ the hydrolysis stage is carried out with stirring by using from 1 to 10 mol of water per mole of amide functional group to be hydrolysed and an amount of strong inorganic acid expressed as protons and corresponding to at least 1 mol of protons per mole of amide to be hydrolysed, the temperature of the reaction medium being determined in order to maintain the reaction medium in the liquid state;
➢ the carboxylic acid formed is recovered by maintaining the reaction medium, without stirring, at a temperature greater than the melting point of the carboxylic acid and/or of the salt formed for separation of the reaction medium by settling, the upper phase comprising the carboxylic acid being separated.

2. Process according to Claim 1, **characterized in that** the amount of water used in the hydrolysis stage is between 1 and 5 mol of water per mole of amide functional group to be hydrolysed, preferably between 1 and 2 mol of water.

3. Process according to either of Claims 1 and 2, **characterized in that** the water necessary for the hydration and hydrolysis reactions is added in the hydration stage according to an amount of water of between 2 and 10 mol of water, preferably from 2 to 3 mol of water, per nitrile functional group to be hydrated.

4. Process according to one of Claims 1 to 3, **characterized in that** the compound comprising nitrile functional groups is chosen from the group consisting of methylbutyronitrile, valeronitrile, 2-methyl-2-butenenitrile, 2-pentenenitrile, 3-pentenenitrile, 4-pentenenitrile, 2-methyl-3-butenenitrile and dinitriles, such as 2-methylbutyronitrile, methylglutaronitrile, ethylsuccinonitrile, succinonitrile, glutaronitrile, adiponitrile or analogous products, and a mixture of at least two of the said compounds.

5. Process according to one of Claims 1 to 4, **characterized in that** the compound comprising nitrile functional groups is a mixture of methylglutaronitrile, ethylsuccinonitrile and adiponitrile.

6. Process according to Claim 5, **characterized in that** the said mixture constitutes the top fraction from the purification or separation of adiponitrile by distillation in the process for the manufacture of adiponitrile by hydrocyanation of butadiene.

7. Process according to either of Claims 5 and 6, **characterized in that** the hydration stage is carried out at a temperature of between 90°C and 180°C and the hydrolysis stage at a temperature of between 90°C and 180°C.

8. Process according to one of Claims 1 to 7, **characterized in that** the inorganic acid is chosen from the group consisting of gaseous hydrochloric acid, phosphoric acid, sulphuric acid and the like.

9. Process according to Claim 8, **characterized in that** the inorganic acid is added in the pure or concentrated form.

10. Process according to one of the preceding claims, **characterized in that** it comprises a stage of esterification of the carboxylic acids present in the upper phase to give esters by reaction with an alcohol.

11. Process according to Claim 10, **characterized in that** the esterification stage is carried out by mixing the separated upper phase with an alcohol and maintaining the reaction medium at a temperature which makes it possible to obtain reflux of the alcohol.

12. Process according to Claim 10 or 11, **characterized in that** the amount of alcohol mixed corresponds to 2 mol of alcohol per mole of diacid.

13. Process according to either of Claims 10 and 12, **characterized in that** a complete esterification of the diacid is carried out by removal of the water formed obtained by continuous addition of alcohol and distillation of a water/alcohol mixture.

14. Process according to one of Claims 10 to 13, **characterized in that** an inorganic acid is added as catalyst.

15. Process according to either of Claims 10 to 14, **characterized in that** the alcohol is chosen from the group consisting of branched or unbranched and cyclic or noncyclic aliphatic alcohols which can comprise an aromatic ring and which can comprise from 1 to 20 carbon atoms.

16. Process according to Claim 15, **characterized in that** the alcohol is chosen from the group consisting of methanol, propanol, isopropanol, benzyl alcohol, ethanol, n-butanol, isobutanol, cyclohexanol, hexanol, isooctanol, 2-ethylhexanol, isomers of pentanols, and isobutanol.

17. Process according to one of Claims 10 to 16, **characterized in that** the esterification reaction is carried out in a reactive column with removal of the water.

18. Process according to one of Claims 10 to 16, **characterized in that** the esterification reaction is carried out by passing the carboxylic compound/alcohol mixture over a sulphonic acid ion-exchange resin.

19. Process according to one of Claims 10 to 18, **characterized in that** the esters formed are recovered and purified by distillation.
